(19) Europäisches Patentamt / European Patent Office / Office européen des brevets

(11) **EP 4 147 749 A1**

(12) **EUROPEAN PATENT APPLICATION**

(43) Date of publication:
**15.03.2023 Bulletin 2023/11**

(21) Application number: **21196623.9**

(22) Date of filing: **14.09.2021**

(51) International Patent Classification (IPC):
***A61N 5/10*** *(2006.01)*

(52) Cooperative Patent Classification (CPC):
**A61N 5/103;** A61N 5/1031

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **BONDAR, Maria Luiza**
**5656 AE Eindhoven (NL)**

• **MARTINUSSEN, Hanneke**
**5656 AE Eindhoven (NL)**
• **WEESE, Rolf Jürgen**
**5656 AE Eindhoven (NL)**
• **NEUKIRCHEN, Christoph**
**5656 AE Eindhoven (NL)**
• **FLAESCHNER, Nick**
**5656 AE Eindhoven (NL)**
• **WIEGERT, Jens**
**5656 AE Eindhoven (NL)**

(74) Representative: **Philips Intellectual Property & Standards**
**High Tech Campus 52**
**5656 AG Eindhoven (NL)**

(54) **MACHINE LEARNING BASED AUTOMATION AND FACILITATION OF VISUAL PLAN REVIEW PROCESS IN RADIOTHERAPY**

(57)     A system (SYS) for computer-assisted plan review in radiation therapy and related method. The system comprises an input interface (IN) for receiving input data comprising a dose distribution image as per a current radiation treatment plan. A classifier module (CM) of the system processes the input data to produce a classification result (q) indicative of a quality assessment for the plan. The system's classification analyzer (CA) produces, based on the classification result and the input data, transparency data (7) indicative as to why the classifier module produced the said classification result. An output interface (OUT) provides output data including the transparency data. The system facilitates plan review and helps reduce time and computational overhead.

FIG. 2

**Description**

FIELD OF THE INVENTION

The invention relates to system for computer assisted radiation therapy plan review, to a training system for training a machine learning model for use in such as system, to a training data generator system, to related methods, to a computer program element, and to a computer readable medium.

BACKGROUND OF THE INVENTION

Cancer is a leading cause of death in industrialized nations around the world. Well over half of cancer patients receive treatment by Radiotherapy ("RT"). In RT, high energy ionizing radiation is applied to the patient, either from outside the patient (external RT) or from within (brachytherapy). The goal is to destroy cancerous tissue whilst preserving non-cancerous, healthy tissue. In some types of external RT, X-radiation in the megavolt (MV) range is used. The total radiation dose is administered in portions (also known as "fractions") over time as per a treatment plan, such as once per day over the course of 2-4 weeks for example.

The preparation of the treatment plan is an important but time-consuming process. A medical dosimetrist may be involved in the planning. In current clinical radiotherapy workflow, a mandatory step is visual evaluation by a radiation oncologist, of a dose distribution associated with the treatment plan. Visual evaluation is required to check that the planned dose distribution matches the treatment intent. In addition, visual evaluation and approval signature by the radiation oncologist are mandatory clinical processes required for department audit. Approval signature indicates that the radiation oncologist is responsible for the treatment.

The process of visual plan evaluation/view is user dependent. Consequently, the quality of this process is user dependent, e.g. the user might miss an important area for visual plan review and approve a bad plan.

Currently, there are no tools to guide and support the user throughout the visual plan evaluation process.

For example, the medical dosimetrist does usual not known whether the plan will fail or pass the visual plan review process.

SUMMARY OF THE INVENTION

There may therefore be a need to address at least some of the above noted limitations and/or to otherwise improve RT workflow.

An objective of the present invention is solved by the subject matter of the independent claims where further embodiments are incorporated in the dependent claims. It should be noted that the following described aspect of the invention equally applies to the to the training system for training the machine learning model, to the training data generator system, to the related methods, to the computer program element, and to the computer readable medium.

According to a first aspect of the invention there is provided a system for computer-assisted plan review in radiation therapy, comprising:

an input interface for receiving input data comprising a dose distribution image as per a current radiation treatment plan;
a classifier module configured to process the input data to produce a classification result indicative of a quality assessment for the plan;
classification analyzer configured to produce, based on the classification result and the input data, transparency data indicative as to why the classifier module produced the said classification result, and
an output interface for providing output data including the transparency data.

In embodiments the transparency data is spatially resolved to provide an indication on how a given region of the dose distribution image contributed to the classification result.

In embodiments, the system includes a graphics display generator, configured to generate, for display on a display device, a graphics display including a visualization of any one of more of i) patient image, ii) the transparency data, and iii) the classification result.

In embodiments, the system includes a user interface configured to allow a user to instruct the graphics display generator to switch between graphic displays to selectively visualize any one or more of i) the image, ii) the transparency data, and iii) the classification result.

In embodiments, the classifier module includes a trained-machine learning model.

In embodiments, the model is of the artificial neural network type.

In embodiments, the transparency data includes a class activation map.

In embodiments, the classifier module is implemented based on an image segmentation algorithm or model.

In embodiments, the system includes a recommender module configured to recommend, based at least on the output data, a new dose distribution or a radiation treatment type.

In yet another aspect there is provided a computer-implemented method for computer-assisted plan review in radiation therapy, comprising:

receiving input data comprising a dose distribution image as per a current radiation treatment plan;

processing the input data to obtain a classification result indicative of a quality assessment for the plan;

producing, based on the classification result and the input data, transparency data indicative as to why the classifier module produced the said classification result, and

providing output data including the transparency data.

The proposed system and method allow reducing time and computational overhead that may otherwise incur in one or more quality check loops of RT planning and plan review. Specifically, a likelihood for re-planning due to a fail in quality check at review can be considerably decreased. The time delay incurred by going (possibly repeatedly) trough replanning/review feedback loops may be reduced for each patient individually, which scales up to considerable time savings when considering the total number of patients that require cancer treatment, a number that is set to increase due to an ageing population in parts of the world. Specifically, the proposed system and method allows user, such as the dosimetrist, to "emulate" review outcomes for a current plan, until a pass is obtained. Only then is the plan forwarded for review to an oncologist for example. In this manner, the number of feedback loops can be reduced. Alternatively, a junior radiation oncologist might use the system to emulate the review by a senior oncologist. As such the system could be used for educational purposes. In addition to, or instead of, review by radiation oncologist, some clinics might wish to hold plan review board meeting for example, where plan(s) are analyzed, and the proposed system may facilitate such analysis.

In another aspect there is provided a training system configured to train the machine learning model based on training data.

In another aspect there is provided a training data generator system configured to generate the training data for use by the training system.

In embodiments, the training data generator system is configured to generate labelled training data based on radiation treatment plan data and response data received from an eye-tracking system operative whilst a reviewer is reviewing the said radiation treatment plan data.

The eye tracking system is of particular benefit for supporting generation of training data for segmentor type model/algorithms, or for classifiers configured to produce localized classification result.

In yet another aspect there is provided a machine learning method for training the machine learning model based on training data.

In yet another aspect there is provided a computer implemented method of generating training data for training the machine learning model.

*"user"* relates to a person, such as medical personnel, clinician, or other, operating the RT delivery equipment, overseeing the RT procedure, drawing up a treatment plan, re-planning or reviewing. The user is in general not the patient.

BRIEF DESCRIPTION OF THE DRAWINGS

Exemplary embodiments of the invention will now be described with reference to the following drawings wherein:

Fig. 1 shows a schematic block diagram of a radiation therapy system;

Fig. 2 shows a block diagram of a radiation therapy planning support system;

Fig. 3 shows a schematic illustration of a graphical user interface as may be used in the system of Fig. 2;

Fig. 4 illustrates training and deployment phases of a machine learning model;

Fig. 5 shows a schematic diagram of a machine learning model of the artificial neural network type;

Fig. 6 shows a training system for training a machine learning model;

Fig. 7 shows a flow chart of a method supporting radiation therapy planning and/or plan review;

Fig. 8 shows a flow chart of a method of generating training data for a machine learning model for supporting radiation therapy planning; and

Fig. 9 shows a flow chart of a method training a machine learning model.

DETAILED DESCRIPTION OF EMBODIMENTS

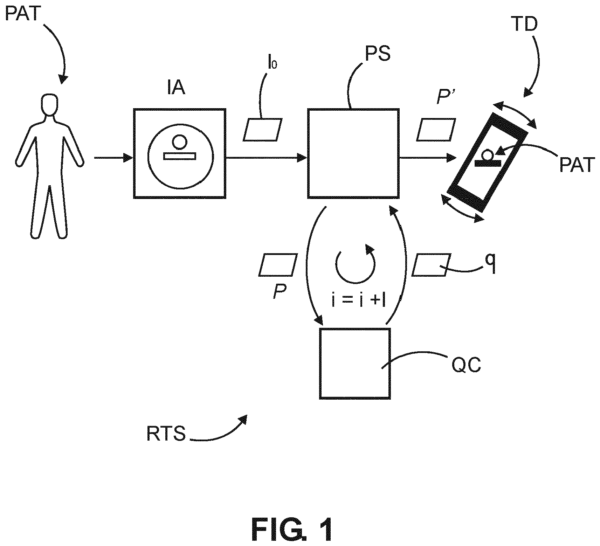

With reference to Fig. 1, there is shown a schematic block diagram of a computerized radiation therapy ("RT")-system RTS. Broadly, the RT system RTS may include a treatment device TD configured for RT delivery based on a treatment plan. The system RTS may further include a treatment planning system PS configured to compute the said plan. Before explaining the new treatment planning system PS in more detail, some context around RT is provided first.

External radiation therapy is mainly envisaged herein although the principles described herein may also be applied to internal radiation therapy. In external radiation therapy, high energy radiation is delivered to a patient to kill off cancerous tissue. The objective is to kill off as much of the cancerous tissue as possible, whilst sparing non-cancerous tissue. The cancer killing radiation is delivered by the treatment device TD such as a linear accelerator ("linac"). In linacs, high energy x-ray photons in the megavolt range are generated and the patient is exposed in a pre-mediated manner to the radiation delivered by the treatment device TD.

Types of X-ray based external RT envisaged herein include intensity-modulated radiation therapy ("IMRT"), volumetric-modulated arc therapy and others. Specifically, in X-ray based external RT, the radiation is delivered at certain doses from a multitude of different spatial directions. More specifically, radiation treatment beams propagating along the said different spatial directions can be made to intersect preferably at the lesion (the target volume TV) to be treated, thus delivering a higher dose there, whilst leaving a lower dose at surrounding non-cancerous tissue, in particular organs at risk (OAR). The applicable doses to be delivered and the spatial directions relative to the lesion site through which the dosages are to be delivered are defined in the treatment plan.

The treatment plan is computed by a planning module PM of the treatment planning system PTS. The planning module PM may be implemented by a computing system. The (final) treatment plan may be the result of the planning module PM engaging with a quality check system QC in one or more loops of replanning and quality-check feedback on earlier versions of the plan. What is proposed herein is an improved planning module, with improved efficiency thanks at least in parts to fewer quality rejections by the quality check system.

Computing the treatment plan is in general a complex mathematical operation that may consume time and computational resources, and hence electrical energy. Preferably, high performance computing processors such as those of multi-core design may be used to secure a reasonable return time.

Computing the treatment plan ("the planning problem"), may be formulated as an optimization. Specifically, one or more objective functions are formulated subject to one or more constraints. The objective function and the constraints define the manner in which the treatment dose is to be delivered given location, shape and extent of the cancerous lesion. More specifically yet, the objective function(s) and the constraint(s) describe how much dosage is to be delivered in which regions. A clinician prescribes for instance the average dose in a certain area, or the maximum or minimum dose to be delivered and formulates these objectives and constraints. The objective function(s) is a 3D scalar field that assign to each spatial point in treatment space, where the patient is located during treatment, a certain deliverable dose. The constraint(s) qualify the deliverable dose. An exemplary setup may be illustrated as an objective to deliver a uniform dose of 60 Gy in 30 fractions to the tumor or target volume TV, with the constraint to spare dose exposure entirely, or at least restrict dose up to a threshold, at certain OARs, such as the parotid gland, the spinal cord, or the healthy lung. A related constraint may for example be formulated as keeping the dose below 25 Gy in more than 70% of the volume of the OAR. The sum of the dose objectives may represent the intended dose. The intended dose may be formulated based on user input, such as provided by a dosimetrist. The planning PM may thus not necessarily operate fully automatic, but may instead include interfaces to implement a user-in-loop setup. The user (for example the dosimetrist) prescribes the dosage objectives by using a graphical user interface (GUI) for example or other type of user interface (UI), to annotate a visualization of a planning imagery representative of the TV and OARs.

The planning imagery is previously acquired by a medical image modality IA, such as a computed tomography scanner CT, a magnetic resonance imager (MRI), or a nuclear imaging modality such as PET/SPECT. Based on this planning imagery 1°, the cancerous region TV may be identified. This initial planning imagery $I^0$ is preferably 3D so as to capture the 3D shape and extent of the cancer lesion situated usually at least partly, if not fully, inside the patient. The target volume TV is identified either manually by delineation in the imagery, or the delineation is done automatically or semi-automatically by a segmentation algorithm. The identified tissue includes the volume TV to be treated where the cancerous tissue resides plus a certain safety margin. Volumes of non-cancerous tissue, such as the above-mentioned OARs, may also be segmented or otherwise identified in the planning imagery. OARs are to be spared from radiation exposure, or deposited radiation is at least not to exceed certain maximum thresholds as formulated in the planning constraints.

The planning module PM implements a planning algorithm to find a solution to the planning problem. In particular, inverse planning algorithms may be iterative and compute how much dosage is to be delivered from which spatial position over a certain period of time. In the iterations, the objective function is improved until an acceptable treatment plan is found. The total dose is deliverable in fractions. In other words, parts of the dose are delivered over a treatment course of days or weeks, in multiple treatment sessions. A treatment session for a fraction is usually once per day. In each such treatment, a certain dose part of the total dose envisaged is delivered from the computed spatial positions.

The RT treatment plan may include in particular a quantity known as a "fluence map". The fluence map φ

defines the intensity of the treatment beam across its cross section. The planning algorithm/technique facilitates computing an optimized fluence map of each lesion of patient. The computing of the fluence map is a computationally complex procedure that involves the mentioned optimization of the objective function. The optimization is constrained to account for the above-mentioned tradeoff between preservation of health issue and killing-off cancer tissue. The computation may be based on the segmented 3D CT image that defines the lesion. The computation of the fluence map amounts to solving a positivity-constrained optimization problem with known methods. See for instance Pflugfelder et αl, "A comparison of three optimization algorithms for intensity modulated radiation therapy", Z. Med. Phys. (2008) Vol. 18, No. 2, pp. 111-119.

Based on the computed fluence map, a machine control program is computed by the planning module PM to control operation of the treatment delivery device TD (such as the linac or other). For example, in IMRT, a leaf sequencing tool computes the control program to control position and timing motions of individual leaves of an MLF collimator of the linac. For instance, in modulated radiation therapy, the treatment device TD includes a treatment head with the said multi-leaf collimator that can realize different beam shapes. The cross section of the beam may be divided into beamlets where different dosages can be realized in a spatially resolved manner. The computed control program of the treatment plan may prescribe different collimator settings that may vary as the treatment head moves to different spatial positions around the target volume. The collimator settings may prescribe how leaf elements of the multi-leaf collimator are to be moved to realize the different beam shapes.

A simulation tool of the planning module PM may process the control program to compute deliverable dose distribution d, if the control program (that is, the current plan) were to be used. The computed dose distribution d may be stored as a spatially resolved file or data structure, also referred to herein as the dose map d.

The dose map d may be transferred via a suitable communication network to the quality checker QC. The quality checker QC may be a fully automated computer system, or may include some user in the loop elements, such as user interface and graphics display generator GDG in communication with a display device DD. The graphics display generator GDG may render the dose distribution map in iso-lines representation. This representation comprises a set of nested, generally closed curves, each representing a dose at a given percentage, and each such curve may circumscribe an area that is to revive at least the said percentage of dose.

The deliverable dose distribution map d may be optionally superimposable on the planning image to represent the dose per location to facilitate a visual quality check by a reviewer (for example, a clinical professional, such as radiation oncologist).

Conducting such a plan quality check by quality checker QC is preferable because the deliverable dose distribution map d as computed by the planning module PM may be at variance with the intended dose as prescribed by the dosimetrist. This variance may be caused by parts of the optimization being ill-posed such that small variations may be amplified into considerable error margins. This may be compounded by errors incurred during the iterative search in solution space, or by solution space including a large number of local minima where the iterative optimization procedure my get trapped, etc. The quality checker QCs may apply one or more metrics to quantify how and to what extent i) the deliverable dose conforms with the TV and ii) the OARs are spared. The metrics may be based on geometric properties of the dose distribution and its relation to the TV and/or OARs. For example, the metrics may include any one or more of:

metric to quantify the extent to which the target is inside a (high) isodose line (e.g., TV is inside the 95% isodose line),
metric to quantify geometric/alignment conformality, that is the extent to which a shape of a high dose isodose lines conforms a shape of the target contour and/or of borders of adjacent AORs,
metric to quantify shape of the dose profile, such as the steepness of the dose- gradient in a region of interest/OAR adjacent to the target TV,
metric to quantify the unevenness of the dose distribution surface such as presence of hot and cold spots, etc, in particular by taking their location relative to TV and/or OAR into account. For example, a cold spot in the target TV close to the spinal cord might be acceptable, and so may be a hot spot in the middle of the target TV, but a hot spot inside an OAR might not be acceptable and attract a low score in relation to this metric,
metric to quantify shape and/or the position of the maximum dose point(s) in the plan/dose distribution,
metric to quantify shape of low isodose lines, as their shape can reveal unexpected dose distributions,

other metrics in terms of statistics of prescribed dose to the targets TA, values of dose metrics to OARs.

Each or some of metrics (1)-(7) or others may be represented/measured in terms of graded scores, that can be graded into a pass or fail. Each metric may be graded separately. An overall pass/fail based on the pass/fails gradings of each metric individually may be awarded.

The metrics may be evaluated automatically by using segmentations for example, and by applying automatic in-image/map measurements. Alternatively, or in addition, the reviewer uses his/her clinical and medical knowledge to award scores for each metric based on visual inspection of displayed dose map. The scores may be stored in a file. The scores may be stored as annotations to the visualized map, etc. The scores are available as an assessment result q.

The assessment result may include an indication of an overall fail or pass score. The fail/pass scores may be awarded for each metric separately, and/or globally, based on some or all the metric scores collectively. The fail/pass score may be indicated graphically or by audio signal or otherwise. A graphics display may include such indications. The graphics display may be displayed on a display device.

The assessment result q may be forwarded to the planning module PM by the or another communication network to inform the dosimetrist. A message or other alert signal may be generated to this effect.

If there is an (overall) pass, the current plan may be forwarded to the radiation oncologist for approval and sign off. The plan may then be used to start the RT. However, if there is a fail, replanning by module PM may be required. Replanning may entail rerunning the planning optimization algorithm as described above, thus consuming extra time/computational resources. Ordinarily, one or more iterations between the planning module and the quality checker may be required in multiple loops at times.

The proposed improved planning module PM is configured to reduce the number of such loops, and preferably get the plan right to pass first time, with improved performance ratio. Computational and time resources could be saved, and patient throughout increased. The improved planning module PM may use classifier module CM. This may be implemented as a suitably trained machine leaning ("ML") model M. The model M is trained in (one or more) training phases on training data. Matters in relation to training and the particular of the mode M will be explored more fully below.

It will be understood that the above-described workflow separation between, on the hand, the dosimetrist at the planning module PM, and the oncologist at the quality checker QC on the other, constitutes an on-purpose "semi-ethics wall"-type of communication protocol arrangement. The dosimetrist and the oncologist should work separately, each coming to their own conclusions. It is in their interaction through the above-described feedback loop that a good plan will ultimately emerge.

Whilst the quality check by the oncologist at the quality checker system QC uses objectively measurable metrics as described above, it is nevertheless the oncologist's medical knowledge and training that ultimately determines the quality assessment result, a fail or a pass. The oncologist considers the deliverable dose distribution over the patient data (medical record etc), planning image and the various metrics in their totality to arrive at the quality assessment result.

In reality, the quality assessments may differ from oncologist to oncologist. It may be said then that some subjective elements are having a bearing to some extent. Having said that, all oncologists will have had medical training so their knowledge, whilst different from individual to individual, collectively may still be considered a common latent factor that drives the assessment results statistically. Similar such domain knowledge factors may be observed on the dosimetrist's side. There is thus a latent relationship between the deliverable dose distributions produced by dosimetrists on the one hand, and the assessment results produced by the oncologist on the other hand. It will be difficult to model this relationship analytically, directly and exactly. Use of machine learning modelling is proposed herein to capture this latent relationship, obviating the need for such explicit, direct, analytical modelling. Instead, historical training data may be used to configure the classifier model M to be capable of predicting, with useful accuracy, likely classification results (in particular, a pass or fail), given a current dose distribution map. The training data for training the predictive classifier model may be readily sourced from available medical databases. The so trained machine learning model may thus be understood as an approximation for the earlier mentioned medical knowledge and the said latent relationship. The machine learning model may be trained on data from various oncologists that capture this general medical knowledge. On the other hand, in embodiments, it may be favorable to train separate machine learning models one for each oncologist on a given site for example. This would allow accounting for certain personal preferences of the various oncologists, to reduce delays introduced by an otherwise unnecessary large number of quality check loops.

The trained machine learning model M may be preferably used by the dosimetrist to estimate whether the current plan P that resulted in the current dose distribution map is likely to attract a pass or fail if it was to be submitted for review by the oncologist. If the classifier model M predicts a fail, the dosimetrist may re-visit the plan model before submitting same for review by oncologist, and make changes. Several such iterations may be done by the dosimetrist at the planning module. The plan may be changed by the dosimetrist once or more times over, and is only submitted to reviewing by the oncologist, if the model M predicts a pass. The more expensive and time-consuming loops between dosimetrist and oncologist can be thus reduced, or possibly avoided altogether, thus yielding a possibly sizable increase in patient throughput.

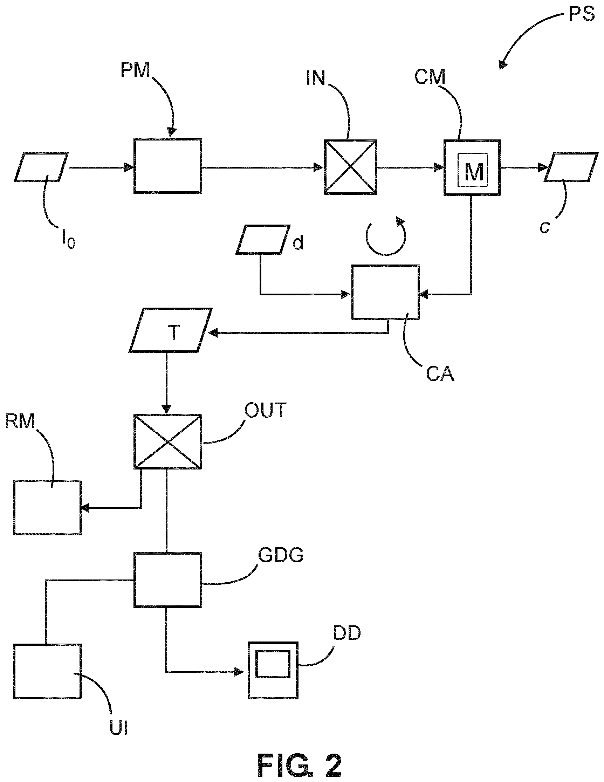

Reference is now made to the block diagram Fig. 2 to explain operation of computerized improved planning support system PS in more detail.

Generally, the system PS may be implemented as suitable software component(s) run by one or more computing unit PU, either general purpose or dedicated. Preferably, the dedicated or general purpose computing system PU may include one or more processors. In particular, one or more multi-core processors such as GPU (graphics processor unit) or TPU (tensor processing unit) may be used. A single one or a plurality of computing units PU may be used, possibly arranged as server(s) communicatively coupled in a communication network such as in a Cloud architecture. In particular, the computing for the machine learning component may be so outsourced to one or more servers. As an alternative to a purely software based implementation, some or all of the components of the system RDS may be implemented as hardware components such as FPGAs or ASICS.

The improved planning system PS may include the above described planning module PM. Planning module PM receives the planning image $I_0$ of a given patient, acquired by a suitable imaging apparatus IA. The imaging apparatus may be configured for X-ray based tomography or radiography. Other modalities, such as nuclear, ultrasound or MRI are also envisaged for imaging apparatus IA. Based on the image $I_0$, associated dose and anatomical constraints, the planning model computes, as described above, a version of the treatment plan P. The treatment plan includes a deliverable dose distribution d obtained by a suitable simulation tool, as outlined earlier above.

The dose distribution d is a scalar field or map. It is therefore apt herein to refer to entries of the map *d* as pixels /or voxel, and treat map *d* much as an image. The grid or voxel size for the map may differ from the voxel size/grid of the planning image. Interpolation may be used for visualization purposes, to match the two grids.

Input Interface IN reads in the deliverable dose distribution map d, to be processed by the trained machine learning model M as described above. The model M computes, based on the distribution map *d*, and on possibly additional contextual input data, a predicted quality assessment. The predicted quality assessment is an estimate for a quality assessment which the deliverable dose distribution map d may have attracted at the quality checker stage QC. The model M may be configured as a classifier to predict a "pass" (P) or a "fail" (F) classification result for the current dose distribution d. The model thus "emulates" a quality check by a human medical expert, such as by an oncologist.

In addition, system PS comprises a quality assessment/classifier analyzer component CA which may or may not be part/component of the machine learning model M. The classification analyzer CA is configured to generate transparency data T, based on the predicted quality assessment $c = P$ or c $=F$. Broadly, the transparency data T includes information that explains (thus makes transparent) how/why the machine learning model arrived at the classification result c.

The transparency data T, also referred to herein as saliency map, measures how the ML arrived at the classification result. A different transparency map $T^c$ is associated with each class c. Broadly, the classification result c is a function of the input data. However, when looked at in more nuance, this statement can be refined. One may ask how the information in the input data d that contributed to the given class c, is spatially spread across the input data. The transparency data answers this question. Different parts of the input d may have contributed differently to the result. The transparency map T is configured to capture this spatial spread of contribution per class c. Spatially, transparency map T corresponds in size/dimension to the input data d. Its entries are modulated in accordance to how the individual dose values contributed to the classification when processed by the ML model M. Whilst the transparency map T may be calculated for each class, it is the transparency map T associated with the fail classification result $c=F$ that is of main interest herein.

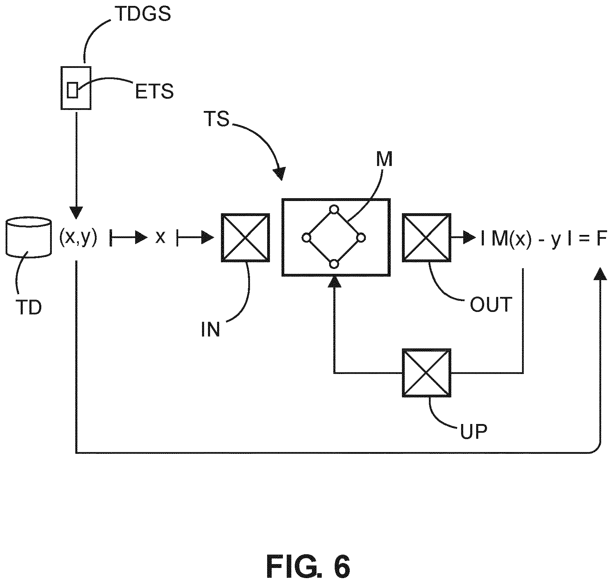

The computation of the transparency data T for machine learning models for the purpose of classification result c interpretability has been described before elsewhere and some of the approaches will be described more fully below. That is, multiple techniques exist to compute the transparency data, any one of which, or a combination thereof, is envisaged herein. Classifier analyzer component CA processes the classification result c and parameters of the trained model M to compute the transparency data T. Very broadly, and in some embodiment. the result c is traced back and across the network M. Contributions of the individual image elements (regions, or pixels/voxel individually) to the result c are ranked by a suitable encoding of elements of the transparency data. Computation of transparency data T will be explained in more detail below at Fig. 6.

As briefly mentioned above, transparency data T is spatial data and has thus image like character. The transparency data T corresponds generally in size and dimension to the input data d, the deliverable dose distribution map. Again, it will be thus apt herein to refer to the above mentioned elements of the transparency data T as pixels or voxels, or regions thereof (subsets of such pixel/voxels). The transparency data T is thus a spatial scalar field, similar to the dose distribution map d. In consistency with what has been observed above in relation to the dose distribution map d, it will thus be apt and more natural to refer to the transparency data set T as a transparency data map T. As observed earlier, there are different transparency data maps $T^c$, one for each classification class, for example one map for fail $c=F$ and one for pass $c= P$, denotable as $T^{c=F}$, $T^{c=P}$, respectively. The transparency map $T^c$ for a give classification result c of interest measures the saliency of elements (pixel/voxel, or per region) in the input distribution map $d$ to the respective result c. Transparency maps $T$ are thus per class c data entities for a given classifier model M.

The transparency map $T$ and the dose distribution map $d$ may be used in combination, for example one superimposed on the other to form a combined image. In addition, the transparency map T may be interpolated on the patient image grid and superimposed on the patient image grid to form a combined image. The transparency map T computed by analyzer CA may be output at output port OUT, for storage or other processing. For example, transparency map T may be used by a graphics display generator GDG to generate a graphics display GD. The transparency map T may be displayed preferably in conjunction with the dose distribution d in the combined image. The combined image may be displayed on a display device DD. The graphics display generator GDG may be preferably used by the dosimetrist when drawing up the current plan P.

The transparency map $T$ allows localizing in dose distribution map $d$, the portions that contributed more than others to the classification result. It is in particular the transparency map $T^{c=F}$ associated with the failed result $c= F$, if any, that is of interest herein and it is this transparency map $T^{c=F}$ that is displayed by graphics display generator GDG, either on its own or on in combination with the input dose distribution d. Preferably the transparency map $T^c$ is thresholded at multiple levels, with levels being color or grey-value coded, to obtain a rendering as a heat map. The heat map indicates regions in the dose distribution map at various saliency levels, including those that attracted the highest saliency level for the respective classification result. A single or multiple saliency levels may be defined. The transparency map $T^c$ may not necessarily be displayed.

The transparency map $T^{c=F}$ may be used instead as control parameters to control or inform replanning of the current plan P, on which more below in relation to recommender module RM. The transparency map $T^c$ may be spatially associated with the dose distribution map d, such as by superposition and association of corresponding spatial coordinates $(x_1, ... ,x_N)$ (with $N$ =2, 3, or of higher dimension if required). The transparency map $T^c$ so spatially associated defines attention regions in the dose distribution map d. The attention regions correspond to regions defined by iso-hypersurfaces (such as isolines or iso(-2D) surfaces). The one or more attention regions indicate spatial features of the dose distribution map that have contributed by at least the given saliency level to the fail classification $c=F$ for example. The attention regions are likely those that would have caused the quality checker stage QC to award the fail result.

The attention regions are likely to center or concentrate around "suspect" regions of the dose distribution map, as mentioned above in relation to the applicable metrics. For example, such attention regions may include regions where not all of the TV receives the prescribed minimum dose level and the radiation oncologist is likely not to accept the loss of TV coverage, or where certain OARs receive more dose than allowed and the radiation oncologist is unlikely to compromise. Other such suspect regions indicated (e.g., are spatially covered) by the attention region(s) may include unfavorably spatial dose distributions, such as cold or hot spots, or those with unduly sharp drop-offs/ peaks, etc. The user can use the classification results and/or the attention regions defined by the transparency map to decide on finalizing the planning process. For example, the user obtains a dose distribution with favorable dose statistics values for TV and OARs. Nonetheless, a common practice among users is to attempt further improvements of the dose distribution, by, for example, attempting to further reduce the dose to regions of healthy tissues or to change the shape of the low dose isolines. The user can decide to stop the planning process after obtaining a pass classification c=P for quality metrics associated to shape of low dose isolines.

The recommender module RM may analyze features indicated by the attention regions to suggest certain changes to the current plan P. The optimization procedure may then be automatically rerun for re-computing a new version P* of the current plan P, using new initial parameters provided by the user. Alternatively, an alert message is displayed for the user, proposing such as rerun and it is the user that triggers, by user input, the said rerun. In the rerun, initial conditions, such as the dosage constraints may be tweaked, or a different type of planning algorithm may be used. The user may use the attention region(s) defined by the transparency map T associated to a failed metric criteria to segment a new region of interest in the planning image $I_0$. The segmentation can be manual or semi-automated (e.g., thresholding with a user-defined value). The user can construct the objective function to further manipulate the dose distribution in the new region of interest. The procedure described above is a semi-automated plan-rerun. However, it might be possible to further manipulate the dose distribution by other means, such as by modifications of weights, type of objective functions associated to the original ROIs (TV or OARs or other). Thus, the information conveyed by the attention region of transparency map $T^{c=F}$ may facilitate replanning.

However, some simpler, embodiments are also envisaged, where there is no such recommender RM. A heat map or a similar visualization of the attention regions of $T^{c=F}$ is caused to be displayed on the display device by graphics display generator GDP. The visual mark up in the graphics display GD as displayed on the display device DD may be sufficient to provide suitable visual clues for the dosimetrist to act on: the dosimetrist may often be able to adjust the planning parameters (such as dose constrains, etc) merely by considering the visualized attention region(s). For example, a visualization of the attention regions superimposed over the current dose distribution map d (as per the current plan P) may be sufficient for the dosimetrist to understand which changes need to be made.

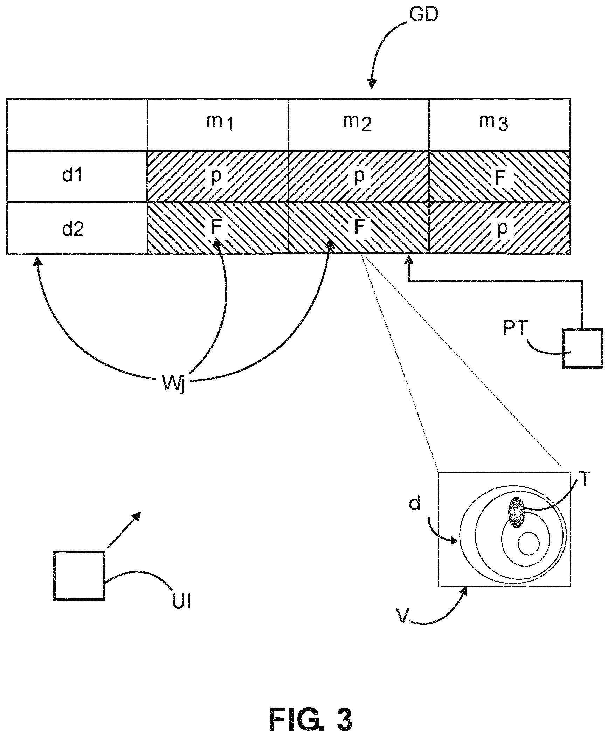

The planning module PM then re-runs the (or a different) planning algorithm to compute the new plan P*, which includes a new dose distribution d', which can then be put again through the machine learning module M to re-emulate the quality assessment, and so forth, in as many iteration loops as desired by the dosimetrist. However, those iterations remain at the dosimetrist's side, so can be done in a much shorter turnaround time because the quality checker QC is not involved at this time. Once the predictor model M indicates a pass c=P, the current plan P* may then be forwarded to the quality checker QC for review. The likelihood that the pass will be confirmed at the quality checker stage QC is thus increased and the overall time delay between planning and commencement of treatment can be reduced. Reference is now made to Fig. 3 which shows an exemplary graphics display GD generatable by the graphics display generator GDG. The graphics display GD may be integrated into a graphical user interface GUI.

As illustrated, the graphics GD may include indicia for the classification results of "pass" (P) and/or "fail" (F) as computed by the trained machine learning classifier model M. The indicia may be textual and /or graphical as illustrated by color or grey value coded widgets $w_j$. Instead of using a single model M, a bank of such machine learning models $M_j$

may be trained, one (or more) for each metric $m_j$, and the respective pass and fail predictions may thus be displayed in the graphic display GD in association with the respective metric $m_j$. However, a simplified graphic display is also envisaged where only a single indication for pass and fail is displayed, representing a global assessment, rather than displaying the individual sub-assessments for the individual metrics as shown in Fig. 3.

A particular embodiment of a suitable GUI layout as envisaged herein in Fig. 3 may include a matrix structure into which the, preferably interactive, widgets $w_j$ are arranged. Rows may indicate the different metrics $m_j$, whilst columns indicate the different dose distributions $d_j$ for different patients for example. A transposed arrangement is also contemplated, and so are GUI arrangements other than in matricial layout.

Each widget wj may include an indication in textual and/or graphical form for the respective predicted quality assessments. Symbol "P" may indicate pass, and symbol "F" may indicate a fail. Other character encodings may be used instead. In addition or instead of character encoding, color/grey value encoding may be used to indicate pass/fail. For example, a green widget may indicate a pass whilst a red widget indicates fail.

The widgets wj are preferably interactive and respond to pointer action by user operable pointer tool PT, such a stylus, computer mouse or other. Touch screen action may be supported instead or in addition. Upon user-initiated activation of a widget wj, a suitable event handler causes the graphics display generator GDG to generate a pop-up window that includes a visualization V of the transparency data T for the class c (= F or $P$) to which the widget relates. The visualization is preferably in combination with the current dose distribution d. The lower right of Fig. 3 shows an exemplary representation of such a visualization, with transparency data T and dose distribution superimposed. The example illustrates the dose distribution in isolines rendition. The shadings correspond to a rendering of the attention regions as encoded by the transparency data T. A color wash visualization scheme may be used for the attention region. Other visualization schemes that allow a concurrent visual appreciation of features of both, the dose distribution and the transparency data T, are also envisaged herein. For example, the dose distribution d is shown overlaid with visualizations of the attention regions. The attention regions are indicative of regions in the dose distribution that contributed more than other regions to the fail classification. In the example illustration of Fig. 3, the fail classification may have been attracted by the feature of not steep enough gradient of the dose distribution, as evidenced by an unduly large a distance between, for example, it's the prescription dose isolines and a percentage of prescription dose isoline.

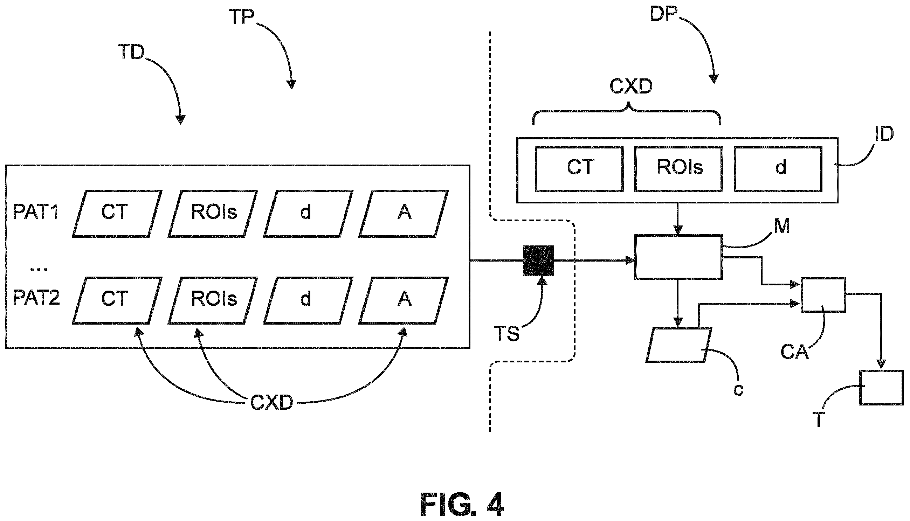

Reference is now made to Fig. 4 which shows a schematic diagram illustrating training phase TP as opposed to deployment phase DP of the machine learning model M. Training data TD is held in a medical database including historical dose distributions $d'$ planned for previous patients PAT$1$-PAT$n$. The medical database may further include additional contextual data CXD, such as the planning images (CT scan or other image type). The historical data may be further include annotations A, or segmentations of ROIs, such as the OARs and the TV. The annotation data A may include the quality assessment results awarded in those historical quality checks.

A training system TS uses the historical data to adapt parameters of a prototype of the model M in a numerical optimization procedure in training phase TP. Some such procedures are gradient based. Some such procedures are iterative. In one or more iterations, which may be controlled by a suitably configured objective function (such as a loss function or utility function), the model parameters are adapted. Once adapted in one or more training phases (which may be a one-off or may be done repeatedly if new training data emerges), the model is deemed sufficiently trained and can be made available for deployment phase DP. In deployment, input data as encountered during clinical practice is received, including the current dose distribution d as explained above, possibly augmented with other contextual data CXD, such as the planning image, etc. The input is processed by the model M to produce the predicted quality assessment result c. The classification analyzer CA computes the transparency data $T$ for classification c. Transparency data T, in particular $T^{c=F}$, allows defining the attention regions as described above. The classification analyzer CA may be an integral part, such as a layer of the machine learning model, but may also be implemented independently as an external entity and is not part of the model as such and is not subjected to the training.

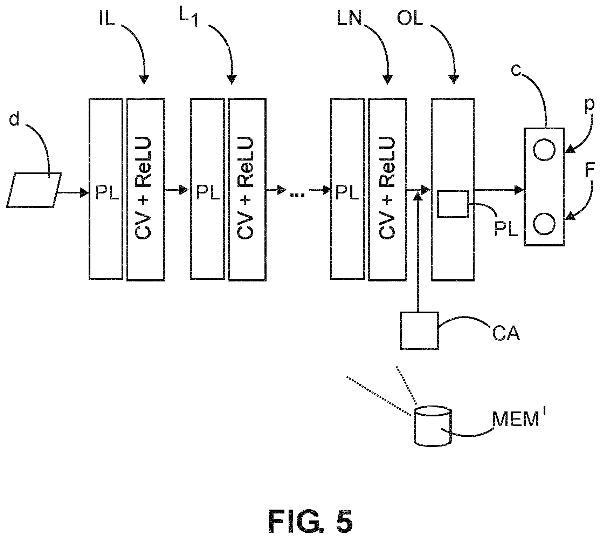

An example model M of the neural network type and operation of classification analyzer CA is now explained in more detail with reference to Fig. 5.

The machine learning model M may be stored in one (or more) computer memories MEM'. The trained model M may be deployed as a machine learning component that may be run on the computing device PU, such as a desktop computer, a workstation, a laptop, etc or plural such devices in a distributed computing architecture. Preferably, to achieve good throughput, the computing device PU includes one or more processors (CPU) that support parallel computing, such as those of multi-core design. In one embodiment, GPU(s) (graphical processing units) are used.

The network M may comprise a plurality of computational nodes arranged in layers in a cascaded fashion, with data flow proceeding from left to right and thus from layer to layer. Recurrent networks are not excluded herein. Convolutional networks have been found to yield good result when processing image data.

In deployment, the input data d is applied to input layer IL. The input data d then propagates through a sequence of hidden layers $L_1$-$L_N$ (only two are shown, but there may be merely one or more than two), to then emerge at an output layer OL as the classification result M(d) = c.

The model network M may be said to have a deep architecture because it has more than one hidden layers.

In a feed-forward network, the "depth" is the number of hidden layers between input layer IL and output layer OL, whilst in recurrent networks the depth is the number of hidden layers, times the number of passes.

Preferably, the hidden layers include a sequence of convolutional layers, represented herein as layers $L_1$ - $L_N$. The number of convolutional layers is at least one, such as 2-5, or any other number. The number may run into double-digit figures.

In embodiments, downstream of the sequence of convolutional layers there may be one or more fully connected layers OL, in particular if a classification result is sought, such as in classification into pass (P)/fail (F). The output layer OL may be configured as a *softmax*-layer. Classification may be binary, but multi-class classification is not excluded herein. The softmax-function of output layer OL normalizes the features maps of the previous layer(s) to obtain the classification scores for P and F. The output may be interpreted as probabilities $p1+p2 =1$, where, say, score $p1$ corresponds to P *and* $p2$ to F.

Some or each hidden $L_m$ layer and the input layer IL implements one or more convolutional operators CV. Each layer $L_m$ may implement the same number of convolution operators CV or the number may differ for some or all layers.

A convolutional operator CV implements a convolutional operation to be performed on its respective input. The convolutional operator may be conceptualized as a convolutional kernel. It may be implemented as a matrix including entries that form filter elements (the said weights) that form at least a part of the model parameters $\theta$. It is in particular these weights that are adjusted in the learning phase.

The first layer IL processes, by way of its one or more convolutional operators, the input data such input dose distribution d. Feature maps are the outputs of convolutional layers, one feature map for each convolutional operator in a layer. The feature map of an earlier layer is then input into the next layer to produce feature maps of a higher generation, and so forth until the last layer OL represents the desired classification result c.

Convolutional filters CV in a given layer process an input feature map from an earlier layer into intermediate output, sometimes referred to as logits. An optional bias term may be applied by addition for example. An activation layer processes in a non-linear manner the logits into the next generation feature map which is then output and passed as input to the next layer, and so forth. The activation layer may be implemented as a rectified linear unit RELU as shown, or as sigmoid-function, *tanh*-function or any other suitable non-linear function.

Conceptually, the neural network model M is a system of interconnected parameters and functions. Gradient-based back-propagation techniques may be used by the classification analyzer CA to ascertain how changes in the classification result c obtained in deployment DP translate into changes across certain nodes/parameters of the model M. In particular the classification analyzer CA analyzes how the responses of the activation layers, including activation units (eg, RELUs) changes with varying result c. This will indicate that certain such nodes/activation units are more important than others, that is, contribute more to the final result than others. Those parameter changes of the model can be back-projected into the initial input data (including the dose distribution d). In other words, to different pixel/voxel positions of the input dose distribution d can be associated a respective measurement score that represents the relative importance/saliency of the information at that location to the final result c. The collection of the so spatially resolved saliency scores form the transparency data T for a given result c.

In other embodiments, class activation techniques are used to compute the transparency data T as class activation maps (CAMs). In such or similar techniques, the classification analyzer CA may comprise a pooling layer PL arranged between the last convolutional layer $L_N$ and the output layer OL. The pooling layers reduces dimension of input feature maps. In order to obtain the CAM, the classification analyzer CA uses the last pooling layer PL to collapse the feature maps of the last layer (before OL) into a vector of numbers, one such number per feature map. Global Average Pooling or Global Max pooling may be used. Global-Average-Pooling produces spatial averages of the feature map of each ReLU in the last convolutional layer. The numbers (in this case a 2-vector) thus obtained are fed into the softmax-final layer OL. The transparency data map (the CAM) may be obtained by projecting-back weights of the output layer on the convolutional feature maps obtained from the last convolution layer Lm. The analyzer CA may produce the CAM as a weighted sum of the feature maps of the last layer. The output so obtained may be up-sampled to match size of input dose map d. Any spatial up-sampling technique may be used, such as bilinear interpolation to obtain the classification transparency map as a CAM. The classification transparency map T may be visualized by superimposing on the input data d (the current dose distribution map). CAM approach has been described by B Zhpu et $\alpha$l in "Learning Deep Features for Discriminative Localization", available as preprint on arXiv:1512.04150v1, Dec 2015. Other approaches for computing transparency maps T, in particular for neural network type models include "Grad-CAM: "Visual Explanations from Deep Networks via Gradient-based Localization" by R R Selvaraju et $\alpha$l, published in 2017 IEEE International Conference on Computer Vision (ICCV), 017, pp. 618-626. Other approaches also envisaged include those based on deconvolution operators, such as proposed by Zeiler et $\alpha$l in "Visualizing and Understanding Convolutional Networks", online preprint arXiv:1311.2901 [cs.CV], Nov 2013. The earlier mentioned gradient-based approaches or similar have been described by K Simonyan et $\alpha$l in "Deep Inside Convolutional Networks: Visualising Image Classification Models and Saliency Maps", online preprint arXiv:1312.6034, April 2014.

It will be understood that the above described model M in Fig. 5 is merely according to one embodiment and is not limiting to the present disclosure. Other neural network architectures are also envisaged herein with more or less or different functionalities than described herein, such as further pooling layers PL, or drop-out layers or others still. What is more, models M envisaged herein are not necessarily of the neural network type. Statistical methods for classification based on sampling from training data are also envisaged herein in alternative embodiments. The model M may be implemented as random forests, a decision tree or as a support vector machine (SVM), or many others still. The classification analyzer CA is adapted to the particular type of model. It is configured to quantify how portions of input d contribute to the output c and this general principle can be applied to any ML model.

Reference is now made to the block diagram of Fig. 6, in order to explain in more detail operation of training system TS to train model M in training phase TP. A bank of such models $M_j$ may be set up and trained separately, one for some or each quality metrics (1)-(8). However, we refer herein generically to a model M which may be understood as a reference to any of the model Mj making up the bank of models.

Whilst the training system TS may operate on historical training data as explained above, such historical training data may need to be labelled first with labels to be used in particular for supervised algorithms as envisaged herein in embodiments. Labelling results in pairs of training data instants $(x_k, y_k)$ which is processable by model M during training. The training data is used to adjust parameters of model M. Each pair k comprises input training data $x_k$ and its associated target $y_k$ ("the label"). For present purposes, the input training data $x_k$ may include historical dose distributions d' and the targets $y_k$ represent pass or fail classifications $c$ '=P or F, awarded in earlier planning/review sessions. Optionally, the input training data is enriched with contextual data $\kappa$ (such as planning image, etc). In this case, there are enriched training data instant which may be written as $([x_k\ \kappa_k], y_k)$.

The labeling may be done manually and explicitly, but is tedious and costly, as it may require review of the training data by clinical professionals such as oncologists to define suitable targets for the training data.

Whilst it may be possible to at least partly automize labelling by using a scripting tool or query scripts based on a database query language, this depends on the availability in the medical data storage of data that can be associated as suitable targets. The scripting tool may search the historical medical records of different patients for their dose distributions d', and information that explicitly or implicitly indicates the classification results c' (the target) awarded back then. For example, the target may be explicitly noted in the records or in metadata such as in header data. etc.

In embodiments, the model M is configured for segmentation. Segmentation algorithms /models M may be considered as a local classifier, instead of a global classifier as mainly envisaged herein and described above. For example, a trained neural network model M awards the pass/fail classification for the whole image. The "localization" may then be done in a postprocessing step by the classification analyzer CA as described above, in particular at Fig. 5, based on the attention regions in the transparency data. Another manner of defining transparency data and attention regions is now described in terms of segmentation, instead of using the post-processing by classification analyzer CA.

The segmentor model M is thus as highly local operator as it operates per voxel/pixel of the dose distribution d itself, or per subsets thereof. Output of the segmentor M may be a binary mask. The binary mask indicates per image element (voxel/pixel) level, which regions of the dose distribution led to the "fail" classification. Instead of a binary mask, a bounding box or other bounding (closed) contour (not necessarily a box) may be output. Again, the bounding contour delineates the region of the dose distribution which was the reason for the "fail" classification. In such embodiments, the output of $M(d)=(c_i\ B,)$, where $c_i$ (i=1,2) is as before the classification result, and $B_i \subset d$ the bounding contour to which the result c, pertains. As before, in case of NN or other ML models, a separate segmentor model Mj may be used per classification task / visual review metric (1)-(8). ML models that deliver so localized classification results and that are not of the segmentor type are also envisaged herein. It may be thus understood that in such model M with classification localization, the classification analyzer is embedded into the model M. The segmentation as proposed herein is for suspicious looking regions in the dose map, such as the ones a human reviewer would have delineated (thus segmented) using a pointer or other segmentation tool. The segmentor model M ss proposed herein in embodiments is trained to segment/delineate like a medically trained human reviewer (eg, oncologist) would. Segmentor model thus processes in deployment input dose map $d$, and outputs as classification result, $M(d) = c = d^s$ a segmented dose map $d^s$, with segments defined therein, representing the above-described attention regions that may have lead to an estimated fail.

For training such a segmentor type models M or models with localized classification more generally, suitable training data may be required in which the training input dose distribution d' not only includes, for example, one binary label per visual review metric, but which may be associated in addition with annotations, such as voxel painting for binary masks or bounding boxes/ other closed contours, etc. In manual embodiment of training data generation, it is the user who generates the labeled training data for example by delineating the regions of the dose distribution which informed his/her review result c, per metric.

Various options are envisaged herein to implement the training system TS. In a preferred embodiment, multitask-learning may be used. In such an approach, a global classification model is trained (as before), with the output being "fail" or "pass" and a second model which is trained for the segmentation. If the models are of the NN type, they may have shared layers / weights to boost training and robustness.

The training scheme may be configured to work even if one has only incomplete annotations/labels at their disposal. For example, there may be more training instances for training the classification network. In this case, the segmentation network is updated only when additionally corresponding delineations $B_i$ are available, or the other way around. Another option is also envisaged, in which only a segmentor model is used. A thresholding may be done: for example, when a certain amount of voxels/pixels are classified as *"fail"*, an overall *"fail'* classification is assigned for the metric currently considered. This would prevent a *"fail"* classification if, e.g., only one of very few voxels/pixels are classified as *"fail"*. In addition, the eye tracker system may be used to more for more efficient labelling/annotation.

With continued reference to in particular the embodiment of the segmentor type model M or other localizing classifiers, in order to make the labelling process for training data compilation more efficient, a training data generating system TDGS may be used in embodiments in cooperation with the training system TS. The raining data generating system TDGS compiles useful instances (pairs) of training data which are accumulated in a training data storge TD. Once a sufficient number of training data has accumulated, this is passed on for processing by the training system TS to train the model M based on the training data. More (new) training data may accumulate for a next training cycle is required.

The training data generating system TDGS may include an eye (movement) tracking system ETS. The eye tracking system ETS may be used during clinical practice by the oncologists to automatically generate the requisite labelling. The oncologist may be asked to wear an eye tracking interface during his/her day-to day reviewing session. Eye movement data of the oncologist during the review process is registered. Such eye movements have been observed to be associable with pass and fail events ultimately rewarded by the reviewer.

The reviewed dose distribution d', its assessment score c', and the eye tracking measurements are stored in association to obtain different instances of training data. This eye tracking scheme using eye tracker ETS may be run on-site for a certain period of time such as weeks or months until enough training data accumulates. It may involve different reviewers at the same site or from different sites to compile a large pool of training data suitably varied to better capture the above-mentioned medical knowledge. Such pool of varied training data may yield high performing models.

Suitable eye (movement) tracking systems (for brevity referred to herein as "eye tracking") have been described before, for example by K Harezlak et $\alpha$I, in "Application of Eye Tracking in Medicine: A Survey", Research Issues and Challenges, Comput. Med Imaging Graph., Issue (65), April, pp 176-190, (2018).

Several technologies for eye tracking exist nowadays and may broadly be grouped into three categories:

a) measurement of the movement of an object (such as a special contact lens) attached to the eye,

b) optical tracking (video-based eye tracking) without direct contact to the eye,

c) measurement of electric potentials using electrodes placed around the eyes.

From the recording of an eye tracker, eye movement elements may be extracted and used to measure the quality of a visual plan review/evaluation. For example, the following two eye movement measurements elements as detected by a video-based eye tracking system ETS may be used herein: i) fixations and ii) saccades. These two types of eye movement may be defined as follows in terms of measurement. A "fixation" may be defined as a moment when a user's eye movements are stable and settled, *"such $\alpha$s in the process of acquiring information from $\alpha$ scene"* (see the *K Harezlak et $\alpha$l.* reference cited above, section 2.5, p 180). The same may apply when user observes an image to extract information therefrom. Fixation may be measured by four attributes: start time, *"duration (about 150-300 ms), x and y coordinates and spatial dispersion of recordings (0.5-2$_\circ$)"* (K Harezlak et al. , Ibid). *"Saccades are very short and fast eye movements towards another fixation location"*. Saccades *"are characterized by durations of (30-80 ms), amplitude (4—20$_\circ$), speed (30—500$_\downarrow$s) and acceleration (4000-8000$_\downarrow$s$^2$)"* (K Harezlak et al. , Ibid). In general, *"there is no visual information gathered during a saccade"* (K Harezlak et al. , Ibid).

Measurements of eye movements can be represented as a map. Based on the above-described measurement attributes, fixations can be distinguished from saccades. For example, a map of attention regions corresponding to fixation measurements can be generated by thresholding the values of the map. The map may be visualized as a heatmap, coded for magnitudes of fixation measurement.

The eye tracking system ETS allows efficiently collecting annotations of regions in the dose image (e.g., region that likely caused a fail result) in addition to binary labels. Application of eye tracking technologies are proposed herein in embodiments to support user to efficiently generate binary labels per fail metric. Preferably, the respective annotations of the dose image d may be collected per metric. Use of the eye tracker ETS as proposed herein is however optional and not mandatory, and the earlier described manners of annotation, and others still, may be used instead or in combination with eye tracking.

Referring now to operation of the training system TS in more detail and with continued reference to Fig 6, in the training phase, an architecture of a machine learning model M, such as the shown CNN network in Fig 5 is pre-populated with initial set of weights. The weights $\theta$ of the model NN represent a parameterization $M^\theta$, and it is the object of the training system TS to optimize and hence adapt the parameters $\theta$ based on the training data $(x_k, y_k)$ pairs. In other words, the learning can be formulized mathematically as an optimization scheme where a cost function $f$ is minimized

although the dual formulation of maximizing a utility function may be used instead.

Assuming for now the paradigm of a cost function $f$, this measures the aggregated residue(s), that is, the error incurred between data estimated by the neural network model NN and the targets as per some or all of the training data pairs $k$:

$$argmin\ _\theta f = \Sigma_k ||M^\theta(x_k) - y_k|| \qquad (9)$$

In eq. (9) and below, function $M_{()}$ denotes the result of the model M applied to input x.

In training, the training input data $x_k$ of a training pair is propagated through the initialized network M. Specifically, the training input $x_k$ for a k-th pair is received at an input IL, passed through the model and is then output at output OL as output training data $M^\theta(x)$. A suitable similarity measure $||\ \bullet\ ||$ is used to measure the difference, also referred to herein as residue, between the actual training output $M^\theta(x_k)$ produced by the model M, and the desired target $y_k$. For classification learning, the measure and the summation in (9) may be formulated in term of cross-entropy or Kullback-Leiber divergence or similar.

The output training *data* $M(x_k)$ is an estimate for target $y_k$ associated with the applied input training image data $x_k$. In general, there is an error between this output $M(x_k)$ and the associated target $y_k$ of the presently considered k-th pair. An optimization scheme such as backward/forward propagation or other gradient based methods may then be used to adapt the parameters $\theta$ of the model M so as to decrease the residue for the considered pair $(x_k, y_k)$ or for a subset of training pairs from the full training data set.

After one or more iterations in a first, inner, loop in which the parameters $\theta$ of the model are updated by updater UP for the currently considered pair $(x_k, y_k)$, the training system TS enters a second, an outer, loop where a next training data pair $x^{k+1}, y^{k+1}$ is processed accordingly. Instead of processing pair by pair, a subset of pairs can be processed at once in batches and the outer loop the processed from batch to batch until the current supply of training data instances is exhausted or until a predefined number of training data instances have been processed, etc. Batchwise processing is preferred.

The structure of updater UP depends on the optimization scheme used. For example, the inner loop as administered by updater UP may be implemented by one or more forward and backward passes in a forward/backpropagation algorithm. While adapting the parameters, the aggregated, for example summed, residues of all the training pairs are considered up to the current pair of for the current batch, to improve the objective function $f$.

The training system as shown in Fig. 6 can be considered for all learning schemes, in particular supervised schemes. Unsupervised learning schemes may also be envisaged herein in alternative embodiments. GPUs may be used to implement the training system TS. The fully trained machine learning module M may be stored in one or more memories MEM' or databases, and can be made available as pre-trained machine learning modules M. The trained model may be made available in a cloud service. Access can either be offered free of charge or their use can be granted via license-pay or pay-per-use scheme.

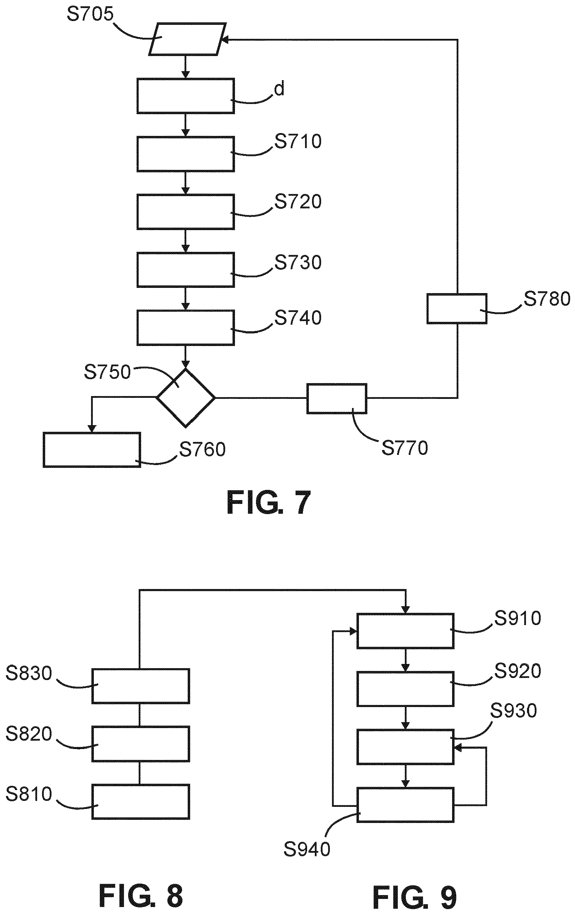

Reference is now made to flow charts in Figs. 7-9. Broadly, the flow chart in Fig. 7 illustrates steps of a computer implemented method for supporting radiation therapy planning. The method may implement the above described planning system PS including the trained machine learning model M and classification analyzer CA, but it will be understood that the below described steps are not necessarily tied to the architecture explained above. That is, the method may be understood as a teaching in their own rights, and so are the additional flow charts in Figs. 8 and 9.

The flow chart in Fig. 7 relates to the deployment phase of the model M once trained, whilst flow charts in Figs. 8-9 relate to the training phase. More specifically, Fig. 8 illustrates steps of a computer implemented method of generating training data for training the machine learning model of the segmentor type, or for those capable of producing localized classification results more generally. The flow chart in Fig. 9 relates to a training method for the machine learning model based on the training data. Whilst the training data generator method in Fig. 8 is preferable, it is not a necessary requirement herein and the machine learning method of Fig. 9 may also be practiced on manually labelled data obtained from historical medical data records, as mentioned above, or one any other suitably sourced or generated training data.

Referring now in more detail to Fig. 7, at step S710 a dose distribution map d for a current plan as computed in a first planning session is received.

At step S720 the plan, in particular dose distribution map d, is processed by a trained machine learning model to produce a classification result. The classification result represents a pass or fail. The computed pass or fail result is an estimate for a quality assessment that a trained oncologist may have awarded if the input dose distribution had been reviewed.

At step S730, jointly with the classification or thereafter, based on the classification result, transparency data is computed. The transparency data is indicative on why the particular classification result in step S720 has resulted. The transparency data may be conceptualized as spatial data, a map, that awards scores to elements of the spatial input data D to measure for element, a contributional importance/saliency for the computed classification result at S720.

The element may include a pixel/voxel, or region(s) or group(s) of pixels/voxels, etc.

At step S740 it is in particular the transparency data that is made available.

At step S750 the classification result at S720 is analyzed. If, at step S750 it is established that the classification result corresponds to a pass, the current plan is forwarded at step S760 for quality check by an oncologist for example.

If, on the other hand, it is established at step S750 that there is a fail awarded at step S720, the transparency data, preferably in combination with the input dose distribution d, may be displayed in a graphics display on a display device.

Alternatively or in addition, the input dose distribution d and its transparency data T for the fail category is otherwise processed to support the planning session.

Specifically, if it is established that a fail has been awarded at step S750, the transparency data associated with fail result is used to change S770 parameters/planning input data of the current planning algorithm. The planning input data may be changed based on the attention regions indicated by the transparency data in the current dose distribution d.

At step S780 a new plan is computed based on the changed planning input data. The new plan P*, including a new dose distribution d*, may then be processed again as described above until a pass is indicated.

Turning now to the training data generation method at Fig. 8, at step S810 it is determined that a visual review process by an oncologist of a current plan including a current dose distribution is conducted.

At step S820, during step S810, eye tracking measurements are obtained with respect to the reviewer reviewing the dose distribution.

At step S830 the eye tracking measurements are associated with the classification result awarded by the reviewer to obtain an automatically labelled instances of training data including annotations in the dose distribution image, in particular in relation to a fail result. Eye tracking is repeated for the same reviewer in multiple reviewing sessions and/or in for other reviewers to build up a pool of training data.

The training data generation method S810-S830 is preferably used for generating annotated versions of dose distribution images/maps that may be used for training segmentation type ML models. The training data generation method S810-S830 may be left out for other ML models, not of the segmentation type. The data generating method may be used during clinical practice by preferably a number of users for period of time until enough training data of suitable variation accumulates.

More detailed reference is now made to flow chart in Fig. 9 which represents steps of a method for training the ML model based on training data, such as the one generated at Fig. 8 when training a segmentor type ML model or other localizing classifier. Other training data, instead of, or in addition to the data generated as per Fig. 8 may be used. Any of the above described manners of collecting training data may be used.

At step S910 training data is received in the form of pairs $(x_k, y_k)$. Each pair includes the training input $x_k$ and the associated target $y_k$. $x_k$, as defined in Fig. 8 above or other.

At step S920, the training input $x_k$ or plural such trainig inputs of a batch is applied to an initialized machine learning model M to produce a training output.

A deviation, or residue, of the training output $M(x_k)$ from the associated target $y_k$ is quantified by a cost function $f$ S930. One or more parameters of the model are adapted at step S940 in one or more iterations in an inner loop to improve the cost function. For instance, the model parameters are adapted to decrease residues as measured by the cost function. The parameters include in particular weights W of the convolutional operators, in case a convolutional NN model M is used.

The training method then returns in an outer loop to step S910 where the next pair of training data or a next batch of such training data pairs is fed in. In step S920, the parameters of the model are adapted so that the aggregated residues of all pairs considered are decreased, in particular minimized. The cost function quantifies the aggregated residues. Forward- backward propagation or similar gradient-based techniques may be used in the inner loop.

More generally, the parameters of the model NN are adjusted to improve objective function $f$ which is either a cost function or a utility function. In embodiments, the cost function is configured to the measure the aggregated residues. In embodiments the aggregation of residues is implemented by summation over all or some residues for all pairs considered. The method may be implemented on one or more general-purpose processing units TS, preferably having processors capable for parallel processing to speed up the training.

The components of the system PS, TS, TDGS may be implemented as one or more software modules, run on one or more general-purpose processing units PU such as a workstation associated with the RT system RTS, or on a server computer associated with a group of such systems.

Alternatively, some or all components of the system PS, TS, TDGS may be arranged in software or in hardware. The hardware may include a suitably programmed microcontroller or microprocessor, such an FPGA (field-programmable-gate-array) or as a hardwired IC chip, an application specific integrated circuitry (ASIC). In a further embodiment still, any one or all of the systems PS, TS, TDGS may be implemented in both, partly in software and partly in hardware.

Any one or all of systems PS, TS, TDGS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1.  A system (SYS) for computer-assisted plan review in radiation therapy, comprising:

    an input interface (IN) for receiving input data comprising a dose distribution image as per a current radiation treatment plan;
    a classifier module (CM) configured to process the input data to produce a classification result (q) indicative of a quality assessment for the plan;

classification analyzer (CA) configured to produce, based on the classification result and the input data, transparency data (*T*) indicative as to why the classifier module produced the said classification result, and an output interface (OUT) for providing output data including the transparency data.

2. System of claim 1, wherein the transparency data (d) is spatially resolved to provide an indication on how a given region of the dose distribution image contributed to the classification result.

3. System of claim 1 or 2, including a graphics display generator (GDG), configured to generate, for display on a display device (DD), a graphics display (GD) including a visualization of any one of more of i) patient image, ii) the transparency data, and iii) the classification result.

4. System of claim 3, including a user interface (UI) configured to allow a user to instruct the graphics display generator (GGD) to switch between graphic displays to selectively visualize any one or more of i) the patient image, ii) the transparency data, and iii) the classification result.

5. System of any one of previous claims, wherein classifier module (CM) is implemented as a trained-machine learning model (M).

6. System of claim 5, wherein the transparency data (*T*) includes a class activation map.

7. System of any one of the previous claims, wherein the classifier module (CM) is implemented based on an image segmentation algorithm.

8. A training system (TS) configured to train the machine learning model of claim 5 based on training data.

9. A training data generator system (TDGS) configured to generate the training data for the training system of claim 8.

10. A training data generator system (TDGS) of claim 11, configured to generate labelled training data based on radiation treatment plan data and response data received from an eye-tracking system (ETS) operative whilst a reviewer is reviewing the said radiation treatment plan data.

11. A computer-implemented method for computer-assisted plan review in radiation therapy, comprising:

receiving (S710) input data comprising a dose distribution image as per a current radiation treatment plan;
processing (S720), by a classifier module, the input data to obtain a classification result (q) indicative of a quality assessment for the plan;
producing (S730), based on the classification result and the input data, transparency data (T) indicative as to why the classifier module produced the said classification result, and
providing (S740) output data including the transparency data.

12. A machine learning method for training the machine learning model of claim 5 based on training data.

13. A computer implemented method of generating training data for training the machine learning model in claim 12.

14. A computer program element, which, when being executed by at least one processing unit, is adapted to cause the processing unit to perform the method as per any one of claims 11-13.

15. At least one computer readable medium having stored thereon the program element of claim 14.

**FIG. 1**

**FIG. 2**

GD

| | m₁ | m₂ | m₃ |
|---|---|---|---|
| d1 | p | p | F |
| d2 | F | F | p |

Wj

PT

UI

d

T

V

**FIG. 3**

**FIG. 4**

EP 4 147 749 A1

**FIG. 5**

**FIG. 6**

S705

d

S710

S720

S730

S740

S750

S760

S770

S780

**FIG. 7**

S830

S820

S810

**FIG. 8**

S910

S920

S930

S940

**FIG. 9**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2018/211725 A1 (PURDIE THOMAS G [CA] ET AL) 26 July 2018 (2018-07-26)<br>* paragraphs [0012], [0017], [0022], [0075] – [0083], [0094], [0102], [0104], [0119] – [0125], [0135]; figures 1,2 *<br>* paragraphs [0175], [0186], [0202], [0203], [0230], [0240], [0289], [0291] * | 1-5,7-9, 11-15<br>6,10 | INV.<br>A61N5/10 |
| X | US 2021/146161 A1 (SINTAY BENJAMIN JEREMIAH [US] ET AL)<br>20 May 2021 (2021-05-20)<br>* paragraphs [0032], [0040] – [0050], [0058] – [0060], [0072]; figure 1 * | 1-9, 11-15 | |
| X | EP 3 486 913 A1 (KONINKLIJKE PHILIPS NV [NL]) 22 May 2019 (2019-05-22)<br>* paragraphs [0040], [0043], [0045], [0046], [0051] – [0058], [0062] *<br>* * | 1-4,7, 11,14,15 | |

TECHNICAL FIELDS
SEARCHED        (IPC)

A61N
G06T

The present search report has been drawn up for all claims

| Place of search | Date of completion of the search | Examiner |
|---|---|---|
| Munich | 17 February 2022 | Link, Tatiana |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 21 19 6623

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2022

| Patent document cited in search report | | Publication date | Patent family member(s) | | Publication date |
|---|---|---|---|---|---|
| US 2018211725 | A1 | 26-07-2018 | CA | 2951048 A1 | 18-12-2014 |
| | | | EP | 3007771 A1 | 20-04-2016 |
| | | | US | 2016140300 A1 | 19-05-2016 |
| | | | US | 2018211725 A1 | 26-07-2018 |
| | | | WO | 2014197994 A1 | 18-12-2014 |
| US 2021146161 | A1 | 20-05-2021 | NONE | | |
| EP 3486913 | A1 | 22-05-2019 | EP | 3486913 A1 | 22-05-2019 |
| | | | US | 2019143147 A1 | 16-05-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PFLUGFELDER.** A comparison of three optimization algorithms for intensity modulated radiation therapy. *Z. Med. Phys.,* 2008, vol. 18 (2), 111-119
- **B ZHPU.** Learning Deep Features for Discriminative Localization. *arXiv:1512.04150v1,* December 2015
- **R R SELVARAJU.** Grad-CAM: "Visual Explanations from Deep Networks via Gradient-based Localization. *IEEE International Conference on Computer Vision (ICCV),* 2017, vol. 017, 618-626
- **ZEILER.** Visualizing and Understanding Convolutional Networks. *arXiv:1311.2901,* November 2013
- **K SIMONYAN.** Deep Inside Convolutional Networks: Visualising Image Classification Models and Saliency Maps. *arXiv:1312.6034,* April 2014
- **K HAREZLAK.** Application of Eye Tracking in Medicine: A Survey. *Research Issues and Challenges, Comput. Med Imaging Graph.,* April 2018, (65), 176-190